(19) **Europäisches Patentamt / European Patent Office / Office européen des brevets**

(11) **EP 1 533 001 B1**

(12) **FASCICULE DE BREVET EUROPEEN**

(45) Date de publication et mention
de la délivrance du brevet:
**07.03.2012 Bulletin 2012/10**

(51) Int Cl.:
***A61N 1/365*** *(2006.01)*

(21) Numéro de dépôt: **04292738.4**

(22) Date de dépôt: **19.11.2004**

(54) **Dispositif médical implantable actif comprenant une fonction de suivi de l'activité sympathico-vagale par analyse de l'accélération endocardiaque**

Aktives medizinisches Implantat mit Überwachung der sympathiko-vagalen Aktivität durch Analyse der endokardialen Beschleunigung

Active implantable medical device monitoring the sympatho-vagal activity by endocardial acceleration analysis

(84) Etats contractants désignés:
**AT BE BG CH CY CZ DE DK EE ES FI FR GB GR HU IE IS IT LI LU MC NL PL PT RO SE SI SK TR**

(30) Priorité: **21.11.2003 FR 0313629**

(43) Date de publication de la demande:
**25.05.2005 Bulletin 2005/21**

(73) Titulaire: **ELA MEDICAL**
**92541 Montrouge (FR)**

(72) Inventeurs:
• **Vitali, Luca**
**10019 Strambino (TO) (IT)**
• **Gaggini, Guido**
**20128 Milano (IT)**

(74) Mandataire: **Dupuis-Latour, Dominique et al**
**SEP Bardehle Pagenberg Dost Altenburg Geissler**
**10, boulevard Haussmann**
**75009 Paris (FR)**

(56) Documents cités:
**EP-A- 0 515 319      EP-A- 0 565 909**
**EP-A- 0 655 260      US-A- 5 609 612**

• **MANGIN L ET AL: "Simultaneous analysis of heart rate variability and myocardial contractility during head-up tilt in patients with vasovagal syncope." JOURNAL OF CARDIOVASCULAR ELECTROPHYSIOLOGY. UNITED STATES JUN 2001, vol. 12, no. 6, juin 2001 (2001-06), pages 639-644, XP009031154 ISSN: 1045-3873**

**Description**

L'invention concerne les dispositifs médicaux implantables actifs tels que définis par la directive 90/385/CE du 20 juin 1990 du Conseil des communautés européennes, notamment les stimulateurs cardiaques, cardioverteurs et/ou défibrillateurs ou dispositifs multisite.

L'invention concerne plus précisément ceux de ces dispositifs qui sont dotés d'une fonction de suivi de l'activité sympathico-vagale du patient porteur de l'appareil implanté.

Le EP-A-0 565 909 (Sorin Biomedica Cardio SpA) décrit un tel dispositif, où l'activité sympathico-vagale est analysée à partir du signal cardiaque recueilli par des électrodes endocavitaires, notamment par analyse de la variabilité des intervalles RR (intervalles séparant deux dépolarisations ventriculaires spontanées consécutives).

L'intérêt du suivi de l'activité sympathico-vagale réside principalement dans la possibilité de prédire la survenue imminente d'une syncope d'origine vaso-vagale et d'empêcher la survenue de cette dernière par une thérapie appropriée, typiquement (mais non exclusivement) une thérapie de stimulation antibradycardique.

Essentiellement, une syncope est une perte de connaissance temporaire avec chute du tonus musculaire, résultant de la réduction momentanée de la circulation cérébrale. Parmi différents types de syncopes, la syncope vaso-vagale est celle provoquée par un déséquilibre temporaire du système de régulation de l'équilibre vaso-vagal, conduisant à une activation excessive du système vagal se traduisant par une vasodilatation et une bradycardie conduisant à la syncope.

Une telle syncope pouvant être anticipée par un certain nombre de signes avant-coureurs, il peut être intéressant chez les patients porteurs d'un stimulateur cardiaque de détecter un risque de survenue imminente d'une telle syncope, pour appliquer une thérapie appropriée et éviter ainsi l'évanouissement du patient.

On considère généralement que la syncope vaso-vagale provient d'un état où le système sympathique présente une réactivité particulièrement élevée qui a pour effet de déclencher une réponse antagoniste, excessive, du système parasympathique provoquant une vasodilatation induisant elle-même la réduction du remplissage des ventricules et la bradycardie.

La chaîne d'événements conduisant à la syncope pouvant commencer plusieurs minutes avant la perte de connaissance, il serait intéressant de pouvoir détecter un tel état le plus tôt possible pour pouvoir prendre toutes mesures appropriées.

Il n'est cependant malheureusement pas possible de mesurer directement l'équilibre du système nerveux autonome. Il est seulement possible d'évaluer cet état de manière indirecte à partir de divers paramètres physiologiques tels que le rythme cardiaque, la contractilité myocardique, l'activité ventilatoire ou d'autres paramètres, ou encore une combinaison de ces divers paramètres.

Les techniques proposées jusqu'à présent se sont cependant révélées difficiles à mettre en oeuvre, compte tenu notamment de la multiplicité des facteurs susceptibles de modifier le rythme cardiaque, facteurs qui ne sont pas nécessairement liés à un déséquilibre du système sympathico-vagal.

La présente invention a pour objet de proposer une autre approche de l'analyse de l'activité sympathico-vagale par un dispositif implanté, en proposant de mettre en oeuvre une détection basée sur l'analyse de l'accélération endocardiaque, plus précisément l'analyse des pics d'accélération endocardiaque.

On sait recueillir un signal d'accélération endocardiaque, comme cela est par exemple décrit dans le EP-A-0 515 319 (Sorin Biomedica Cardio SpA) qui décrit une sonde endocavitaire pourvue d'une électrode distale de stimulation implantée au fond du ventricule, intégrant en outre un micro-accéléromètre permettant de mesurer l'accélération endocardiaque.

Le signal d'accélération endocardiaque ainsi mesuré au cours d'un cycle cardiaque forme, entre autres, deux pics correspondant aux deux bruits majeurs qu'il est possible de reconnaître dans chaque cycle d'un coeur sain.

Le EP-A-0 655 260 (Sorin Biomedica Cardio SpA) décrit une manière de traiter le signal d'accélération endocavitaire délivré par le capteur situé en bout de sonde pour en dériver, notamment, ces deux valeurs de pic d'accélération endocardiaque, utiles notamment pour la détection de troubles cardiaques et le déclenchement ou non d'une thérapie de défibrillation.

Il a été proposé par Mangin et coll., "Simultaneous Analysis of Heart Rate Variability and Myocardial Contractility During Head-Up Tilt in Patients with Vasovagal Syncope", Journal of Cardiovascular Electrophysiology, Vol. 12, 6, 639-644, June 2001, d'utiliser cette information dans le domaine temporel pour analyser l'activité sympathico-vagale du patient.

Cet article décrit un dispositif selon le préambule de la revendication 1.

L'invention propose un mode particulier, exposé dans la partie caractérisante de la revendication 1, d'évaluation de l'activité sympathico-vagale du patient, à partir des moyennes à long terme et des moyennes à court terme des valeurs des pics d'accélération endocardiaque relevées au cours d'une pluralité de cycles successifs et de la fréquence cardiaque.

En effet, le premier pic d'accélération endocardiaque ("PEA I") correspond à la fermeture des valvules mitrale et tricuspide, au début de la phase de contraction ventriculaire isovolumique (systole). Les variations de ce premier pic sont étroitement liés aux variations de la pression dans le ventricule (l'amplitude du pic PEA I étant, plus précisément, corrélée au maximum positif de la variation de pression dP/dt dans le ventricule gauche) et peuvent donc constituer un paramètre représentatif de la contractilité du myocarde, elle-même liée au niveau d'activité du système sympathique.

Le second pic d'accélération endocardiaque ("PEA II"), quant à lui, correspond à la fermeture des valvules aortique et pulmonaire, au moment de la phase de relaxation ventriculaire isovolumique. Ce second pic, qui est produit par la décélération brusque de la masse sanguine en mouvement dans l'aorte, constitue un paramètre représentatif de la pression sanguine périphérique au début de la diastole.

Les sous-revendications visent des caractéristiques subsidiaires avantageuses, avec notamment mise en oeuvre de moyens de détection de risque de syncope, permettant de commander des moyens d'application d'une thérapie préventive et/ou pour produire un signal d'alerte.

On va maintenant décrire plus en détail l'invention, en référence à la figure unique annexée qui est un chronogramme montrant les variations de l'accélération endocavitaire ainsi que de l'électrogramme et de l'électrocardiogramme de surface correspondants, au cours de trois cycles cardiaques successifs.

Sur la figure, on a représenté (courbe du haut), les variations de l'accélération endocardiaque (EA), mesurée par un capteur tel que celui décrit dans le EP-A-0 515 319 précité, intégré à une tête de sonde endocavitaire placée au fond du ventricule. On a également illustré sur cette figure les tracés de l'électrogramme (EGM), c'est-à-dire du signal électrique recueilli par l'électrode distale de ce même capteur, et d'un électrocardiogramme de surface (ECG) correspondant, au cours de trois cycles cardiaques consécutifs.

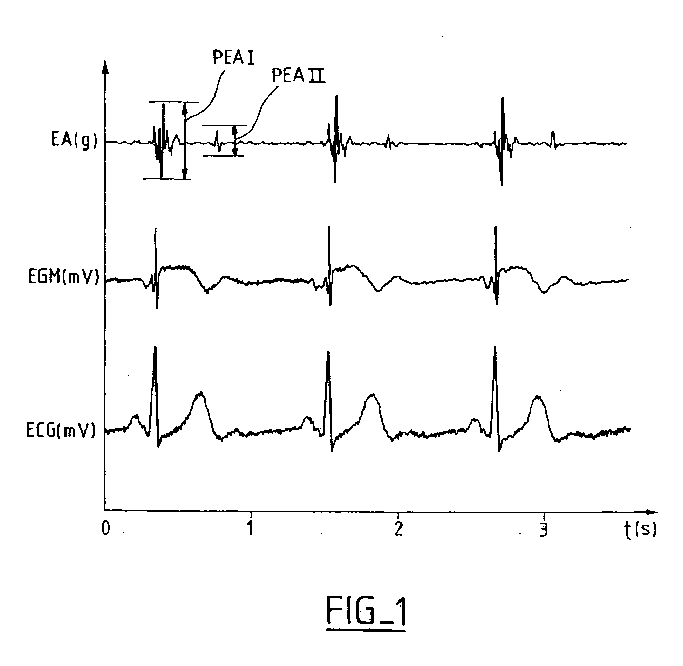

Comme on l'a expliqué plus haut l'accélération endocardiaque passe par deux pics successifs, dont l'amplitude peut être déterminée par un traitement approprié du signal délivré par le capteur d'accélération, comme décrit dans le EP-A-0 655 260 précité. Par "pic" on entendra la valeur maximale crête-à-crête du signal d'accélération séparant les deux extrema, positif et négatif, correspondant aux écarts PEA I et PEA II indiqués sur le chronogramme de la figure 1.

Ces valeurs de pics PEA I et/ou PEA II sont relevées au cours de cycles successifs et analysées pour détecter une situation de risque de survenue imminente d'une syncope vaso-vagale. On notera que l'analyse peut être appliquée au signal PEA I seul, au signal PEA II seul, ou bien à une combinaison des deux signaux PEA I et PEA II.

Ces paramètres (PEA I et/ou PEA II) peuvent être traités de diverses manières.

Une première technique consiste à déterminer, cycle à cycle, les valeurs absolues que prennent ces paramètres et à fixer des seuils de déclenchement d'alarme - ou, de préférence, déterminer une valeur moyennée de ces paramètres sur un nombre prédéterminé de cycles, pour éviter les influences de la variabilité cycle-à-cycle (dispersion des mesures) et celles des événements brefs non significatifs.

Pour améliorer la spécificité de la détection, et notamment pour tenir compte des différences de valeur de base des paramètres PEA d'un individu à l'autre, il peut être avantageux d'analyser les variations de ces paramètres plutôt que leurs valeurs absolues.

Une manière de procéder consiste à analyser la différence entre une moyenne à court terme et une moyenne à long terme du même paramètre. Si ce paramètre varie peu, la différence sera faible et les deux valeurs finiront par coïncider. En revanche, dès que le paramètre deviendra instable, la moyenne à court terme va suivre les variations du paramètre plus rapidement que la moyenne à long terme. La différence entre les deux moyennes ne sera plus nulle ou quasi-nulle, mais prendra une valeur positive (en cas d'augmentation du paramètre) ou négative (en cas de diminution), la valeur absolue de cet écart dépendant du paramètre analysé et de la vitesse de variation de celui-ci.

Pour décider de la présence ou non d'un risque imminent de syncope, un ou plusieurs seuils sont fixés, et chacun des paramètres PEA I ou PEA II, (ou une combinaison de ces deux paramètres) est comparé à un seuil prédéterminé. Le résultat de la comparaison peut être combiné de diverses manières avec le résultat de comparaisons semblables d'autres paramètres pour produire un signal de sortie à deux états, l'un des états étant associé à une situation normale et l'autre état étant associé à une alerte de risque de syncope.

On donnera dans l'exemple plus bas des détails de mise en oeuvre d'une telle technique.

Il est également possible d'utiliser un processus de type "machine à états", où les résultats des comparaisons aux divers seuils sont appliqués à un système à transition d'états et à mémoire, qui prend la décision de déclencher une alerte de risque de syncope en fonction d'un schéma d'évolution plus complexe.

D'autres types d'analyses, plus complexes, peuvent également être mises en oeuvre pour améliorer encore la qualité du procédé de détection, par exemple des techniques de corrélation, d'analyse de morphologie du signal, d'analyse fréquentielle, d'analyse par ondelettes, etc.

Le procédé de détection peut également prendre en compte non seulement les paramètres PEA I et/ou PEA II, mais également d'autres paramètres tels que la fréquence cardiaque, ou encore des signaux délivrés par un capteur d'activité, un capteur de ventilation-minute, etc.

On peut également prévoir que le système soit auto-adaptatif, c'est-à-dire qu'il puisse s'adapter à des variations sur le long terme ou qu'il puisse déterminer si, après avoir produit un signal d'alerte de syncope, une syncope s'est effectivement produite ou non, afin d'améliorer ultérieurement la spécificité du système de détection.

*Exemple*

On va maintenant décrire un exemple d'algorithme de détection basé sur l'analyse combinée du premier pic d'accélération (PEA I), du second pic d'accélération (PEA II) et de la fréquence cardiaque.

Ces trois quantités sont mesurées à chaque cycle cardiaque et un algorithme calcule, pour chacune d'entre elles, deux moyennes glissantes, à long terme et à court terme, ces moyennes étant mises à jour régulièrement (à chaque cycle, tous les quatre cycles, ou tous les dix cycles, etc.).

L'algorithme détermine ainsi les six quantités suivantes :

$PEA1_{LT}$ : moyenne glissante à long terme (par exemple sur 1000 cycles) du paramètre PEA I,
$PEA1_{CT}$ : moyenne glissante à court terme (par exemple sur 30 cycles) du paramètre PEA I,
$PEA2_{LT}$ : moyenne glissante à long terme (par exemple sur 1000 cycles) du paramètre PEA II,
$PEA2_{CT}$ : moyenne glissante à court terme (par exemple sur 30 cycles) du paramètre PEA II,
$FC_{LT}$ : moyenne glissante à long terme (par exemple sur 5000 cycles) de la fréquence cardiaque,
$FC_{CT}$ : moyenne glissante à court terme (par exemple sur 100 cycles) de la fréquence cardiaque.

Pour déterminer le risque de survenue d'une syncope, l'algorithme évalue les trois quantités booléennes suivantes.

$$( PEA2_{CT} < k_1.PEA2_{LT} ) \ \& \ ( FC_{CT} > k_2.FC_{LT} ) \qquad (1)$$

$$( PEA1_{CT} < k_3.PEA1_{LT} ) \ \& \ ( FC_{CT} > k_4.FC_{LT} ) \qquad (2)$$

$$( PEA2_{CT} < k_5.PEA2_{LT} ) \ \& \ ( FC_{CT} > k_6.FC_{LT} ) \qquad (3)$$

La condition (1), si elle est vérifiée, indique que bien que la fréquence cardiaque augmente au-dessus de sa valeur de base, le paramètre PEA II diminue - c'est-à-dire que la pression sanguine diastolique périphérique décroît, correspondant à une situation anormale.

La condition (2), si elle est vérifiée, indique que bien que la fréquence cardiaque augmente au-dessus de la valeur de base, le paramètre PEA I décroît - c'est-à-dire que la contractilité myocardique diminue, révélant une baisse d'activité du système sympathique, situation ici encore considérée comme anormale.

La condition (3), qui correspond à la condition (1) mais avec des critères plus stricts, révèle si elle est vérifiée un état relativement proche de la syncope, lorsque le système nerveux autonome n'est plus en mesure de réguler la stabilité de la pression sanguine.

Un exemple d'application numérique pour les facteurs $k_1$ à $k_6$ est :

$$( PEA2_{CT} < 0{,}75.PEA2_{LT} ) \ \& \ ( FC_{CT} > 1{,}25.FC_{LT} ) \qquad (1)$$

$$( PEA1_{CT} < 0{,}95.PEA1_{LT} ) \ \& \ ( FC_{CT} > 1{,}25.FC_{LT} ) \qquad (2)$$

$$( PEA2_{CT} < 0{,}55.PEA2_{LT} ) \ \& \ ( FC_{CT} > FC_{LT} ) \qquad (3)$$

Diverses mises en oeuvre peuvent être envisagées en variante ou en complément du mode d'analyse donné ci-dessus en exemple. Il est notamment possible d'évaluer le risque de survenue d'une syncope à partir d'une analyse de l'énergie contenue dans le signal d'accélération endocardiaque au niveau du pic PEA I et/ou du pic PEA II, ou encore par une analyse de ce même signal d'accélération endocardiaque, telle qu'une analyse temps/fréquence ou une analyse de l'aire sous la courbe de ce signal, ou encore une analyse de la largeur du pic.

**Revendications**

1. Un dispositif médical implantable actif, notamment un stimulateur cardiaque, défibrillateur, cardioverteur et/ou dispositif multisite ou un implant actif à visée purement diagnostique, comprenant :

   - des moyens de recueil de l'accélération endocardiaque du patient, et
   - des moyens d'analyse, recevant en entrée ladite accélération endocardiaque recueillie et délivrant en sortie des données représentatives de l'activité sympathico-vagale du patient,
   ces moyens d'analyse étant aptes à délivrer en sortie au moins une valeur fonction de l'un et/ou de l'autre de deux pics de ladite accélération endocardiaque au cours d'un cycle donné, ces deux pics comprenant un premier pic (PEA 1) lors de la phase de contraction ventriculaire isovolumique et un second pic (PEA II) lors de la phase de relaxation ventriculaire isovolumique,

   dispositif **caractérisé en ce que** les moyens d'analyse sont des moyens aptes à délivrer en sortie des données fonction :

   - d'une moyenne à long terme et d'une moyenne à court terme des valeurs des premiers pics d'accélération endocardiaque (PEA I) relevées lors de la phase de contraction ventriculaire isovolumique au cours d'une pluralité de cycles successifs ;
   - d'une moyenne à long terme et d'une moyenne à court terme des valeurs des seconds pics d'accélération endocardiaque (PEA II) relevées lors de la phase de relaxation ventriculaire isovolumique au cours d'une pluralité de cycles successifs ; et
   - d'une moyenne à long terme et d'une moyenne à court terme des valeurs de fréquence cardiaque.

2. Le dispositif de la revendication 1, comprenant en outre des moyens de détection de risque de syncope, recevant en entrée lesdites données représentatives délivrées par les moyens d'analyse, et délivrant en sortie un signal d'alerte en cas de risque avéré de survenue imminente d'ùne syncope.

3. Le dispositif de la revendication 2, dans lequel les moyens de détection comprennent des moyens pour produire ledit signal d'alerte lorsque, à un facteur de proportionnalité près, la moyenne à court terme des valeurs des seconds pics d'accélération est inférieure à la moyenne à long terme des valeurs des seconds pics d'accélération et la moyenne à court terme des valeurs de fréquence cardiaque est supérieure à la moyenne à long terme des valeurs de fréquence cardiaque, pour un pourcentage donné de cycles sur un nombre prédéterminé de cycles successifs.

4. Le dispositif de la revendication 2, dans lequel les moyens de détection comprennent des moyens pour produire ledit signal d'alerte lorsque, à un facteur de proportionnalité près, la moyenne à court terme des valeurs des premiers pics d'accélération est inférieure à la moyenne à long terme des valeurs des premiers pics d'accélération et la moyenne à court terme des valeurs de fréquence cardiaque est supérieure à la moyenne à long terme des valeurs de fréquence cardiaque, pour un pourcentage donné de cycles sur un nombre prédéterminé de cycles successifs.

5. Le dispositif de la revendication 2, comprenant en outre des moyens d'application d'une thérapie préventive, opérant en réponse à la délivrance dudit signal d'alerte par modification d'un paramètre de fonctionnement et/ou déclenchement d'une fonction du dispositif.

6. Le dispositif de la revendication 2, dans lequel les moyens de détection comprennent des moyens pour comparer lesdites données représentatives à un seuil prédéterminé et produire un signal d'alerte au franchissement de ce seuil.

7. Le dispositif de la revendication 2, dans lequel les moyens de détection comprennent une machine à états apte à comparer lesdites données représentatives à une pluralité de seuils prédéterminés, à détecter le franchissement des différents seuils, à analyser la séquence de ces franchissements et à produire un signal d'alerte sur détection d'une ou plusieurs séquence(s) de franchissement prédéterminées.

8. Le dispositif de la revendication 2, dans lequel les moyens d'analyse comprennent des moyens pour appliquer au paramètre d'accélération endocardiaque un traitement d'autocorrélation, d'analyse morphologique, d'analyse fréquentielle et/ou d'analyse par ondelettes.

9. Le dispositif de la revendication 2, dans lequel lesdits moyens d'analyse sont des moyens aptes à délivrer également en sortie une valeur fonction de la fréquence cardiaque du patient.

**Claims**

1. Active implantable medical device, especially a pacemaker, defibrillator, cardioverter and/or multisite device or an active implant with purely diagnostic intent, comprising:

   - means for gathering the endocardial acceleration of the patient, and
   - analysis means, receiving as input the said gathered endocardial acceleration and delivering as output data representative of the sympatho-vagal activity of the patient,
   these analysis means being able to deliver as output at least one value dependent on one and/or on the other of two peaks of the said endocardial acceleration in the course of a given cycle, these two peaks comprising a first peak (PEA I) during the isovolumic ventricular contraction phase and a second peak (PEA II) during the isovolumic ventricular relaxation phase,

   device **characterized in that** the analysis means are means able to deliver as output data dependent on:

   - a long-term average and a short-term average of the values of the first endocardial acceleration peaks (PEA I) logged during the isovolumic ventricular contraction phase in the course of a plurality of successive cycles,
   - a long-term average and a short-term average of the values of the second endocardial acceleration peaks (PEA II) logged during the isovolumic ventricular relaxation phase in the course of a plurality of successive cycles, and
   - a long-term average and a short-term average of the values of heart rate.

2. Device of Claim 1, furthermore comprising means for detecting risk of syncope, receiving as input the said representative data delivered by the analysis means, and delivering as output an alert signal in the case of substantiated risk of imminent occurrence of a syncope.

3. Device of Claim 2, in which the detection means comprise means for producing the said alert signal when, to within a proportionality factor, the short-term average of the values of the second acceleration peaks is lower than the long-term average of the values of the second acceleration peaks and the short-term average of the values of heart rate is higher than the long-term average of the values of heart rate, for a given percentage of cycles out of a predetermined number of successive cycles.

4. Device of Claim 2, in which the detection means comprise means for producing the said alert signal when, to within a proportionality factor, the short-term average of the values of the first acceleration peaks is lower than the long-term average of the values of the first acceleration peaks and the short-term average of the values of heart rate is higher than the long-term average of the values of heart rate, for a given percentage of cycles out of a predetermined number of successive cycles.

5. Device of Claim 2, furthermore comprising means for applying a preventive therapy, operating in response to the delivery of the said alert signal, by modifying an operating parameter and/or triggering a function of the device.

6. Device of Claim 2, in which the detection means comprise means for comparing the said representative data with a predetermined threshold and producing an alert signal upon the crossing of this threshold.

7. Device of Claim 2, in which the detection means comprise a state machine able to compare the said representative data with a plurality of predetermined thresholds, to detect the crossing of the various thresholds, to analyse the sequence of these crossings and to produce an alert signal on detection of one or more predetermined crossing sequence(s).

8. Device of Claim 2, in which the analysis means comprise means for applying an autocorrelation processing, morphological analysis processing, frequency analysis processing and/or wavelet analysis processing to the endocardial acceleration parameter.

9. Device of Claim 2, in which the said analysis means are means able to also deliver as output a value dependent on the heart rate of the patient.

**Patentansprüche**

1. Aktive implantierbare medizinische Vorrichtung, insbesondere ein Herzstimulator, ein Defibrillator, ein Kardioverter und/oder eine Multisite-Vorrichtung oder ein aktives Implantat mit rein diagnostischer Zielsetzung, die Folgendes umfasst:

   - Mittel zum Erfassen einer endokardialen Beschleunigung des Patienten und
   - Analysemittel, die als Eingang die erfasste endokardiale Beschleunigung empfangen und als Ausgang Daten ausgeben, die die sympathiko-vagale Aktivität des Patienten repräsentieren,
   wobei diese Analysemittel als Ausgang wenigstens einen Wert liefern können, der eine Funktion der einen und/oder der anderen von zwei Spitzen der endokardialen Beschleunigung im Verlauf eines gegebenen Zyklus ist, wobei diese zwei Spitzen eine erste Spitze (PEA I) in der isovolumetrischen ventrikulären Kontraktionsphase und eine zweite Spitze (PEA II) in der isovolumetrischen ventrikulären Relaxationsphase umfassen,

   wobei die Vorrichtung **dadurch gekennzeichnet ist, dass** die Analysemittel Mittel sind, die als Ausgang Daten liefern können, die eine Funktion sind:

   - eines langfristigen Mittelwerts und eines kurzfristigen Mittelwerts der Werte der ersten endokardialen Beschleunigungsspitzen (PEA I), die in der isovolumetrischen ventrikulären Kontraktionsphase während mehrerer aufeinander folgender Zyklen abgetastet werden;
   - eines langfristigen Mittelwerts und eines kurzfristigen Mittelwerts der Werte der zweiten endokardialen Beschleunigungsspitzen (PEA II), die in der isovolumetrischen ventrikulären Relaxationsphase während mehrerer aufeinander folgender Zyklen abgetastet werden; und
   - eines langfristigen Mittelwerts und eines kurzfristigen Mittelwerts der Werte der Herzfrequenz.

2. Vorrichtung nach Anspruch 1, die außerdem Mittel zum Detektieren eines Synkopenrisikos umfasst, die als Eingang die repräsentativen Daten empfangen, die von den Analysemitteln geliefert werden, und als Ausgang ein Warnsignal in dem Fall liefert, in dem die Gefahr eines bevorstehenden Eintritts einer Synkope entsteht.

3. Vorrichtung nach Anspruch 2, wobei die Detektionsmittel Mittel umfassen, um das Warnsignal zu erzeugen, wenn für einen gegebenen Anteil von Zyklen bei einer vorgegebenen Anzahl aufeinander folgender Zyklen bis auf einen Proportionalitätsfaktor der kurzfristige Mittelwert der Werte der zweiten Beschleunigungsspitzen kleiner ist als der langfristige Mittelwert der Werte der zweiten Beschleunigungsspitzen und der kurzfristige Mittelwert der Werte der Herzfrequenz größer ist als der langfristige Mittelwert der Werte der Herzfrequenz.

4. Vorrichtung nach Anspruch 2, wobei die Detektionsmittel Mittel umfassen, um das Warnsignal zu erzeugen, wenn für einen gegebenen Anteil von Zyklen bei einer vorgegebenen Anzahl aufeinander folgender Zyklen bis auf einen Proportionalitätsfaktor der kurzfristige Mittelwert der Werte der ersten Beschleunigungsspitzen kleiner ist als der langfristige Mittelwert der Werte der ersten Beschleunigungsspitzen und der kurzfristige Mittelwert der Werte der Herzfrequenz größer ist als der langfristige Mittelwert der Werte der Herzfrequenz.

5. Vorrichtung nach Anspruch 2, die außerdem Mittel zum Anwenden einer vorbeugenden Therapie umfasst, die in Reaktion auf die Lieferung des Warnsignals durch Modifikation eines Betriebs- und/oder Auslöseparameters einer Funktion der Vorrichtung arbeitet.

6. Vorrichtung nach Anspruch 2, wobei die Detektionsmittel Mittel umfassen, um die repräsentativen Daten mit einem vorgegebenen Schwellenwert zu vergleichen und um bei Überschreiten dieses Schwellenwerts ein Warnsignal zu erzeugen.

7. Vorrichtung nach Anspruch 2, wobei die Detektionsmittel eine Zustandsmaschine umfassen, die die repräsentativen Daten mit mehreren vorgegebenen Schwellenwerten vergleichen, die Überschreitung der verschiedenen Schwellenwerte detektieren, die Sequenz dieser Überschreitungen analysieren und ein Warnsignal bei Detektion einer oder mehrerer vorgegebener Überschreitungssequenzen erzeugen kann.

8. Vorrichtung nach Anspruch 2, wobei die Analysemittel Mittel umfassen, um auf den endokardialen Beschleunigungsparameter eine Autokorrelations-verarbeitung, eine Verarbeitung der morphologischen Analyse, eine Häufigkeitsanalyseverarbeitung und/oder eine Verarbeitung der Analyse durch Wavelets anzuwenden.

9. Vorrichtung nach Anspruch 2, wobei die Analysemittel Mittel sind, die außerdem als Ausgang einen Wert liefern können, der eine Funktion der Herzfrequenz des Patienten ist.

FIG_1

## RÉFÉRENCES CITÉES DANS LA DESCRIPTION

### Documents brevets cités dans la description

- EP 0565909 A
- EP 0515319 A
- EP 0655260 A

### Littérature non-brevet citée dans la description

- **Mangin.** Simultaneous Analysis of Heart Rate Variability and Myocardial Contractility During Head-Up Tilt in Patients with Vasovagal Syncope. *Journal of Cardiovascular Electrophysiology,* Juin 2001, vol. 12 (6), 639-644